Europäisches Patentamt

(19) European Patent Office     (11) Publication number: **0 138 499**

Office européen des brevets     **B1**

(12)    **EUROPEAN PATENT SPECIFICATION**

(45) Date of publication of patent specification: **29.03.89**     (51) Int. Cl.⁴: **G 01 N 25/04**, G 01 N 33/20

(21) Application number: **84306698.6**

(22) Date of filing: **01.10.84**

(54) Molten metal sampler with tellurium additive.

<table>
<tr><td>(30) Priority: <b>03.10.83 US 538149</b></td><td>(73) Proprietor: <b>Falk, Richard Arden<br>519 Westminster Drive<br>Waukesha Wisconsin53186 (US)</b></td></tr>
<tr><td>(43) Date of publication of application:<br><b>24.04.85 Bulletin 85/17</b></td><td>(72) Inventor: <b>Falk, Richard Arden<br>519 Westminster Drive<br>Waukesha Wisconsin53186 (US)</b></td></tr>
<tr><td>(45) Publication of the grant of the patent:<br><b>29.03.89 Bulletin 89/13</b></td><td></td></tr>
<tr><td>(84) Designated Contracting States:<br><b>AT BE DE FR GB IT SE</b></td><td>(74) Representative: <b>Fisher, Bernard et al<br>Raworth, Moss & Cook 36 Sydenham Road<br>Croydon Surrey CR0 2EF (GB)</b></td></tr>
<tr><td>(56) References cited:<br><b>GB-A-1 038 483<br>US-A-3 946 594<br>US-A-4 261 740</b></td><td></td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to a molten metal sampler cup in which a sample of molten iron is intended to be treated with tellurium to enable determination of the solidus temperature and the carbon content of the sample. Various prior art patents, such as U.S. patents 4,059,996 and 4,261,740 discuss the placement of quantities of tellurium in sample cups for the purpose of delaying graphite formation so that the cooling curve of a hypereutetic iron sample can be obtained to provide a usable liquidus arrest temperature for carbon determination.

In the prior art, in addition to the use of blobs of material containing tellurium, the prior art also includes use of a wash of tellurium which covers the inside walls of the crucible and bottom of the crucible. These prior art techniques do not involve the use of a standardised amount of tellurium or a consistent location of the tellurium in each sample cup. As a result, non-uniform samples were produced and the test results were not always representative or comparable.

GB-A-1,038,483 discloses a molten metal sampler cup having a ceramic body with an opening in its base through which extends a temperature sensor in the form of a thermocouple. The thermocouple is surrounded by a protective tube which upstands from the base of the cup. An external connector structure is provided to allow the thermocouple to be electrically connected to a temperature measuring circuit. No special provision is made for the addition of tellurium in the cup.

According to the present invention there is provided a molten metal sampler cup comprising wall means defining a receptacle for molten metal, the receptacle having a base and an external connector structure and a protective tube for a heat sensing element upstanding from the base to the interior of the receptacle, characterised in that a cast annular tellurium body containing a predetermined amount of tellurium is disposed about and located by the protective tube and secured within the receptacle at a location spaced from said wall means and from said base.

For a better understanding of the invention and to show how the same may be carried into effect, reference will now be made, by way of example, to the accompanying drawings, in which:-

Fig. 1 is a perspective view of one embodiment of a tellurium body being formed in place in a molten metal sampler cup;

Fig. 2 is an enlarged sectional view of the sampler cup of Fig. 1;

Fig. 3 is a perspective view of the tellurium body and a heat sensor;

Fig. 4 is a sectional view of another embodiment;

Fig. 5 is a perspective view of a moulding dish illustrated in Fig. 4;

Fig. 6 is a sectional view of another embodiment;

Fig. 7 is a perspective view of a tellurium body shown in Fig. 6; and

Fig. 8 is a sectional view along lines 8-8 of Fig. 7.

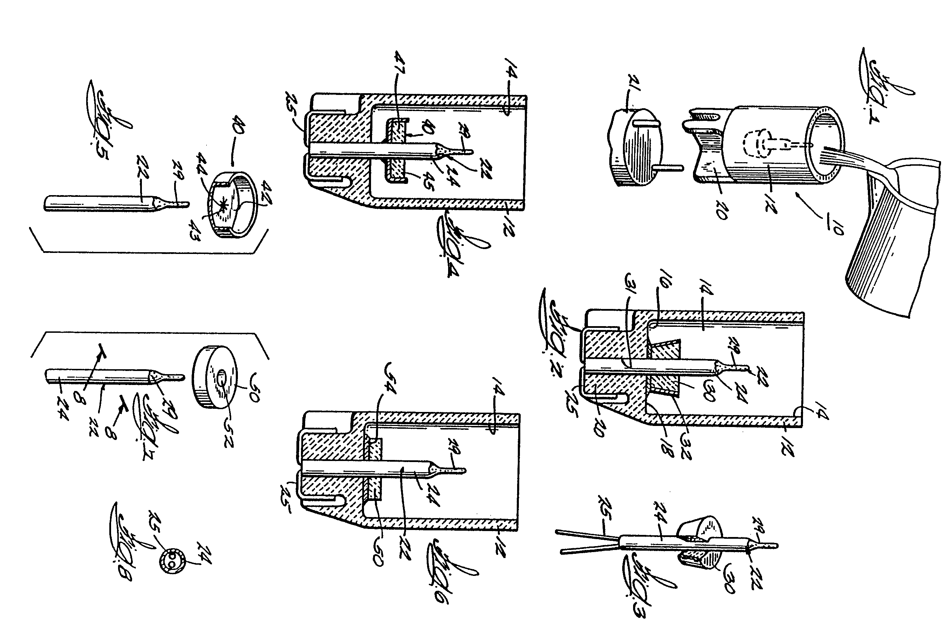

Fig. 1 shows a sampling cup 10 which can be moulded from foundry sand and which includes wall means 12 defining a cylindrical cavity 14 for receiving a molten metal sample. Wall means 16 also form a floor 18. A connector structure 20 is provided which is connectable to an electrical connector 21 which provides electrical connection to the heat sensor or thermocouple unit 22 contained within a temperature resistant glass or ceramic tube 24. In the disclosed construction, the tube 24 is formed from ceramic material with two spaced insulated wires 25 (Fig. 8). The hot junction of the wires is protected by a coating 29 of ceramic cement. The tube 24 extends into the cavity 14 through an aperture 31. The tube 24 is anchored in the aperture 31 in concentric or centred relationship with respect to wall 12. This results in positively positioning the tellurium casting in the centre of the cavity 14, as hereinafter described.

In the construction disclosed in Figs. 1, 2 and 3, a frusto-conical tellurium casting 30 is moulded in place around the tube 24 by pouring a predetermined amount of tellurium together with sand and binder mix into a frusto-conical mould 32 which is arranged around the tube 24. The mould can be formed of a thin member such as tape. Alternatively, the frusto-conical casting can be premoulded and placed in assembly with the tube 24, and the assembly can then be cemented in place by refractory cement.

Where the tellurium body 30 is cast in situ about the protective tube, it is preferred that the mould be formed of a material which is consumable by the molten metal of the sample to which it is exposed in the use of the sampler cup.

Figs. 4 and 5 show a modified embodiment in which the mould is a dish 40 provided with an aperture 42 having radial slits 43 with flexible segments 44 which can be pushed over the tube 24 to provide a seal and tight fit on the tube 24. The tellurium mix can then be poured in the dish cavity 45 to form the insert 47 (Fig. 4). In this example the tellurium body 47 is spaced from the floor 18 of the cavity 14.

Figs. 6 and 7 show a further modified embodiment in which a pre-cast washer or disc 50 with a pre-formed central aperture 52 is moulded from the tellurium sand and binder mix and then inserted around the tube 24 and connected to the floor 18 of the cavity (14) and spaced from it by a layer of refractory cement 54.

The tellurium body in all embodiments described is of circular cross-section and of lesser diameter than the cavity 14 with which it is coaxial.

In all embodiments, significant portions of the tellurium casting are located or spaced from the floor 18 and the cylindrical wall of the cavity 14. This ensures exposure of the tellurium to the molten iron. A tellurium coating or blob on the floor or wall of the cup, as in prior art devices, is readily covered by a skin of iron, chilled by the walls or bottom of the cup, and hence the tellurium is not readily and completely vapourized or dispersed throughout the molten metal to provide uniformity in the sample of hypereutetic iron necessary for measurement of carbon content.

With the Fig. 4 embodiment, the tellurium mix is spaced a significant distance off the floor 18. This will ensure that the tellurium is not covered over by a metal skin, which can cause transfer of the tellurium vapour through the walls of the mould cavity rather than into the sample. In addition, in all embodiments the tellurium mix is retained in place during gasification. The upwardly tapered wall 32 (Fig. 1) in which the largest cross-section of the tellurium body is remote from the base prevents the tellurium from floating out of place.

**Claims**

1. A molten metal sampler cup (10) comprising wall means (12) defining a receptacle for molten metal, the receptacle having a base (18) and an external connector structure (20) and a protective tube (24) for a heat sensing element upstanding from the base (18) to the interior of the receptacle, characterised in that a cast annular tellurium body (30, 47, 50) containing a predetermined amount of tellurium is disposed about and located by the protective tube (24) and secured within the receptacle at a location spaced from said wall means (12) and from said base (18).

2. A sampler cup according to claim 1, characterised in that the tellurium body is cast in situ about the protective tube.

3. A sampler cup according to claim 2, characterised in that the tellurium body is cast in a mould (32) having a central aperture configured so as positively to locate and secure the mould on the protective tube (24).

4. A sampler cup according to claim 2 or 3, characterised in that the tellurium body is cast in situ about the protective tube in a mould of material consumable by the molten metal of the sample to which it is exposed in use of the sampler cup.

5. A sampler cup according to any one of the preceding claims, characterised in that the cast tellurium body (30) is of frusto-conical shape with its largest cross-section remote from the base (18).

6. A sampler cup according to claim 1, characterised in that the cast tellurium body is in the form of a disc (50) with a central aperture (52) dimensioned to receive the protective tube (24).

7. A sampler cup according to any one of the preceding claims, characterised in that the base (18) is formed with an aperture (31) through which the protective tube (24) extends into the receptacle.

8. A sampler cup according to any one of the preceding claims, characterised in that the heat sensing element is a thermocouple within the protective tube (24) and electrically connected to the connector structure (20).

9. A sampler cup according to any one of the preceding claims, characterised in that the interior of the receptacle is of circular cross-section and the tellurium body is axially located within the receptacle by the protective tube.

10. A sampler cup according to any one of the preceding claims, characterised in that the tellurium body is cemented to the base of the interior of the receptacle and spaced from it by a layer of refractory cement (54).

**Patentansprüche**

1. Metallschmelze-Probenehmerschale (10) Metallschmelze, mit Wandmitteln (12), die ein Gefäß für die Metallschmelze definieren, wobei das Gefäß einen Boden (18) und eine Außenanschlußkonstruktion (20) und ein Schutzrohr (24) für ein Wärmefühlerelement hat, das vom Boden (18) in das Innere des Gefäßes hochragt, dadurch gekennzeichnet, daß ein ringförmiger Tellur-Gußkörper (30, 47, 50), der eine vorherbestimmte Menge Tellur enthält, rund um das Schutzrohr (24) angeordnet und durch dieses festgelegt sowie innerhalb des Gefäßes an einer Stelle in Abstand von den Wandmitteln (12) und vom Boden (18) befestigt ist.

2. Probenehmerschale nach Anspruch 1, dadurch gekennzeichnet, daß der Tellurkörper in situ um das Schutzrohr herum gegossen ist.

3. Probenehmerschale nach Anspruch 2, dadurch gekennzeichnet, daß der Tellurkörper in einer Form (32) gegossen ist, die eine mittlere Öffnung hat, die derart gestaltet ist, um die Form am Schutzrohr (24) formschlüssig festzulegen und zu befestigen.

4. Probenehmerschale nach Anspruch 2 oder 3, dadurch gekennzeichnet, daß der Tellurkörper in situ um das Schutzrohr in einer Form aus einem Material gegossen ist, das von der Metallschmelze der Probe, der es beim Gebrauch der Probenehmerschale ausgesetzt ist, abschmelzbar ist.

5. Probenehmerschale nach einem der vorhergehenden Ansprüche, dadurch gekennzeichnet, daß der Tellur-Gußkörper (30) eine kegelstumpfförmige Gestalt hat, wobei der größte Querschnitt vom Boden (18) abgelegen ist.

6. Probenehmerschale nach Anspruch 1, dadurch gekennzeichnet, daß der Tellur-Gußkörper in Form einer Scheibe (50) mit einer mittleren Öffnung (52) vorliegt, die bemessen ist, um das Schutzrohr (24) aufzunehmen.

7. Probenehmerschale nach einem der vorhergehenden Ansprüche, dadurch gekennzeichnet, daß der Boden (18) mit einer Öffnung (31) ausgebildet ist, durch die sich das Schutzrohr (24) in das Gefäß erstreckt.

8. Probenehmerschale nach einem der vorhergehenden Ansprüche, dadurch gekennzeichnet, daß das Wärmefühlerelement ein Thermoelement innerhalb des Schutzrohres (24) und mit elektrischer Verbindung mit der Anschlußkonstruktion (20) ist.

9. Probenehmerschale nach einem der vorhergehenden Ansprüche, dadurch gekennzeichnet, daß das Innere des Gefäßes einen kreisförmigen Querschnitt hat und der Tellurkörper axial innerhalb des Gefäßes durch das Schutzrohr festgelegt ist.

10. Probenehmerschale nach einem der vorhergehenden Ansprüche, dadurch gekennzeichnet, daß der Tellurkörper durch eine Schicht aus feuerfestem Zement (54) an den Boden des Inneren des Gefäßes und in Abstand hievon zementiert ist.

## Revendications

1. Coupe échantillon de métal fondu (10) comprenant une paroi (12) délimitant un receptacle pour le métal fondu, le réceptacle comportant une base (18) et une structure de connexion extérieure (20) et, un tube protecteur (24) pour un élément sensible à la chaleur s'étendant vers le haut à partir de la base (18) vers l'intérieur du réceptacle, caractérisée en ce que un corps annulaire en tellure moulé (30, 47, 50) contenant une quantité prédéterminée de tellure est disposé autour, et protégé par le tube protecteur (24) et est fixé à l'intérieur des réceptacles en un emplacement espacé de ladite paroi (12) et de ladite base (18).

2. Coupe échantillon selon la revendication 1 caractérisée en ce que le corps de tellure est moulé in situ autour du tube protecteur.

3. Coupe échantillon selon la revendication 2, caractérisée en ce que le corps de tellure est moulé dans un moule (32) présentant une ouverture centrale configurée de manière à loger et fixer le moule sur le tube protecteur (24).

4. Coupe échantillon selon la revendication 2 ou 3 caractérisée en ce que le corps de tellure est moulé in situ autour du tube protecteur dans un moule en un matériau qui peut être consumé par le métal fondu de l'échantillon auquel il est exposé lors de l'utilisation de la coupe échantillon.

5. Coupe échantillon selon l'une quelconque des revendications précédentes, caractérisée en ce que le corps de tellure moulé (30) est de forme tronconique avec une section droite plus importante éloignée de la base (18).

6. Coupe échantillon selon la revendication 1 caractérisée en ce que le corps de tellure moullé présente la forme d'un disque (50) avec une ouverture centrale (52) qui est dimensionnée de manière à recevoir le tube protecteur (24).

7. Coupe échantillon selon l'une quelconque des revendications précédentes caractérisée en ce que le tube (18) est pourvu d'une ouverture (31) au travers de laquelle le tube protecteur (24) s'étend dans le réceptacle.

8. Coupe échantillon selon l'une quelconque des revendications précédentes caractérisée en ce que l'élément sensible à la chaleur est un thermocouple à l'intérieur du tube protecteur (24) et qui est connecté électriquement à la structure de connexion (20).

9. Coupe échantillon selon l'une quelconque des revendications précédentes, caractérisée en ce que l'intérieur du réceptacle présente une section droite circulaire et le corps de tellure est positionné axialement à l'intérieur du réceptacle, par le tube protecteur.

10. Coupe échantillon selon l'une quelconque des revendications précédentes caractérisée en ce que le corps de tellure est collé sur la base de l'intérieur du réceptacle et est espacé de cette dernière, par une couche de ciment refractaire (54).

EP 0 138 499 B1

Fig.1
Fig.2
Fig.3
Fig.4
Fig.5
Fig.6
Fig.7
Fig.8

1